(19) Europäisches Patentamt
European Patent Office
Office européen des brevets

(11) **EP 2 811 906 B1**

(12) **EUROPÄISCHE PATENTSCHRIFT**

(45) Veröffentlichungstag und Bekanntmachung des Hinweises auf die Patenterteilung:
**24.04.2019 Patentblatt 2019/17**

(21) Anmeldenummer: **13703577.0**

(22) Anmeldetag: **07.02.2013**

(51) Int Cl.:
**A61B 5/16** (2006.01)   **G06F 3/01** (2006.01)

(86) Internationale Anmeldenummer:
**PCT/EP2013/052484**

(87) Internationale Veröffentlichungsnummer:
**WO 2013/117673 (15.08.2013 Gazette 2013/33)**

(54) **VERFAHREN UND EINRICHTUNG ZUR REKONSTRUKTION VON INTENDIERTEN AKTIVITÄTEN AUS NEURONALEN SIGNALEN**

METHOD AND DEVICE FOR RECONSTRUCTING INTENDED ACTIVITIES FROM NEURAL SIGNALS

PROCÉDÉ ET SYSTÈME POUR LA RECONSTRUCTION D'ACTIVITÉS PRÉVUES À PARTIR DE SIGNAUX NEURONAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.02.2012 DE 102012100999**

(43) Veröffentlichungstag der Anmeldung:
**17.12.2014 Patentblatt 2014/51**

(73) Patentinhaber: **CorTec GmbH**
**79110 Freiburg (DE)**

(72) Erfinder: **RICKERT, Jörn**
**79106 Freiburg (DE)**

(74) Vertreter: **2s-ip Schramm Schneider Bertagnoll Patent- und Rechtsanwälte Part mbB**
**Postfach 86 02 67**
**81629 München (DE)**

(56) Entgegenhaltungen:
**US-A1- 2003 078 522   US-A1- 2010 094 097**

**Beschreibung**

Gebiet der Erfindung

Die Erfindung betrifft ein Verfahren und eine Signalverarbeitungseinrichtung zur Rekonstruktion von Repräsentationen neuronaler Signale.

Stand der Technik und Hintergrund der Erfindung

Gedachte (intendierte) Aktivitäten wie Bewegungen, Sprache, Emotionen oder bildliche Vorstellungen können mit Hilfe neurobiologischer Methoden aus der Gehirnaktivität eines Menschen abgeleitet und annäherungsweise rekonstruiert werden. Aus dem Stand der Technik bekannte Detektionssysteme erlauben die räumlich-zeitliche Erfassung von Gehirnaktivitäten eines Menschen, um daraus Signale abzuleiten, welche die gedachte Bewegung, Sprache, Emotion oder bildliche Vorstellung repräsentieren. Beispielsweise können Folienelektroden oder Tiefenelektroden vorgesehen sein, welche bevorzugt mit einer hohen zeitlichen Auflösung, etwa im Bereich zwischen 1 Hz und 1.000 Hz, die Gehirnaktivitäten erfassen und als elektrische Signale zur weiteren Verarbeitung bereitstellen.

Bekannte Verfahren und Methoden zur Rekonstruktion von Bewegungen, Sprache, etc. aus Gehirnaktivitäten sind in "Brain-Machine Interface Engineering (Synthesis Lectures on Biomedical Engineering); Justin C. Sanchez, et al.; Morgan & Claypool Publishers; 1. Auflage, November 2006", "Toward Brain-Computer Interfacing (Neural Information Processing); Guido Dornhege et al.; The MIT Press; 1. Auflage, Juli 2007" und "Brain-Computer Interfaces: An international assessment of research and development trends; Theodore W. Berger et al.; Springer Netherlands; 1. Auflage, November 2008" beschrieben.

Mit den aus dem Stand der Technik bekannten Vorrichtungen zur Ableitung von Gehirnaktivitäten werden diese gemessen und an eine Auswerteeinheit, etwa ein Rechnersystem übergeben. Hierzu werden standardmäßig Großgeräte wie Magnetresonanztomographen (MRT) verwendet, welche die lokale Gehirnaktivität indirekt durch den Verbrauch von Sauerstoff messen (funktionelles MRT). Ferner können auch elektrophysiologische Geräte verwendet werden, die die lokalen Gehirnströme messen (Elektroencephalographie, Elektrocorticographie, Messung von lokalen Feldpotenzialen oder Messung von Einzelzellaktivitäten mittels intracorticaler Elektroden), verstärken und dann an die Auswerteeinheit senden.

Die Rekonstruktion erfolgt durch ein Modell zur Übersetzung der gemessenen Gehirnaktivitäten in Bewegungen, Laute/Wörter, Emotionen oder Bilder (im Weiteren zusammenfassend Zustände oder Aktivitäten genannt], welches in der Regel in der Auswerteeinheit arbeitet. Das Modell ist dabei in der Regel ein mathematischer Algorithmus, der die gemessenen Gehirnaktivitäten mit Hilfe einer Datenverarbeitungseinrichtung (Computer und/ oder Embedded-System) echtzeitnah in Zustände/Aktivitäten umrechnet.

Für die Generierung des Modells sind verschiedene Verfahren bekannt. Beispielsweise können aus der Signalanalyse bekannte Verfahren, wie etwa "Reverse Correlation" oder die Anpassung von, meist linearen Filtern zur Generierung des Modells verwendet werden.

Die Modelle können einerseits die Funktion der gemessenen Gehirnaktivitäten widerspiegeln, oder naiv, ohne Annahmen über die Funktion der gemessenen Gehirnaktivitäten, durch Verknüpfung dieser mit verschiedenen vorgegebenen Trainingsreizen berechnet werden, oder durch eine Kombination aus beidem erstellt werden. Am Ende steht das Modell als ein Übersetzer für die gemessenen Gehirnaktivitäten in die jeweils ausgewählten Zustände/Aktivitäten.

Nachteilig bei den aus dem Stand der Technik bekannten Rekonstruktionsverfahren ist die hohe Unschärfe bzw. Ungenauigkeit der Rekonstruktionsergebnisse. Diese hat verschiedene Gründe, die sich mit den bekannten Methoden nicht beheben lassen:
Mit der funktionellen Magnetresonanztomographie kann zwar die Aktivität in allen Bereichen des Gehirns gemessen werden, allerdings nur mit einer sehr schlechten zeitlichen Auflösung im Bereich von Sekunden - während das Gehirn im Bereich von Millisekunden oder darunter arbeitet. Die räumliche Auflösung der Gehirnaktivität ist zwar gut und wird ständig verbessert, allerdings besitzt sie auch mit den besten Geräten und Methoden eine Größenordnung von einem Kubikmillimeter - während die funktionellen Strukturen im Gehirn eine Größenordnung von wenigen Mikrometern besitzen.

Ähnliche Unzulänglichkeiten weisen auch die elektro-physiologischen Messmethoden auf. Dort wird - auch bei Messungen mit einer hohen Zahl von Elektroden (mehrere hundert) - nur ein Bruchteil der Aktivität des Gehirns von ausgewählten Arealen aufgezeichnet. Sämtliche Messungen können technisch bedingt also nur unvollständig Gehirnaktivitäten erfassen.

Hinzu kommt die Ungenauigkeit der Messung. Gehirnaktivitäten weisen sehr geringe Stromflüsse auf die unter schwierigen Bedingungen (im lebenden Gewebe in einem bewegenden Körper) gemessen werden müssen. Dies hat zur Folge, dass die Signale nicht exakt gemessen werden können und in der Regel mit Artefakten kontaminiert sind.

Als weiterer Faktor für die Ungenauigkeit bzw. Unschärfe der Messungen kommt hinzu, dass die Gehirnaktivität bei ein und demselben Reiz unterschiedlich sein kann. Dieses Phänomen wird entweder als intrinsisches Rauschen ("neuronal noise") oder als physiologisch relevante Aktivität aufgrund der andauernden und parallelen Verarbeitung verschiedener Zustände (vgl. "Dynamics of ongoing activity: explanation of the large variability in evoked cortical responses; Arieli A. et al.; Science, 27. September 1996, Seiten 1868 - 1871") bezeichnet.

Diese Merkmale der Erfassung von Gehirnaktivität führen dazu, dass diese immer mit einer Ungenauigkeit/Unschärfe behaftet sind. Im Ergebnis erhält man beispielsweise Korrelationskoeffizienten im Bereich von 0,5 für die Rekonstruktion von Bewegungen oder Dekodierungsgenauigkeiten für Vokale im Bereich von lediglich 80%.

US2010/0094097 A1 betrifft ein Verfahren zum Ausführen einer Aktion gemäß dem Oberbegriff von Anspruch 1. US2003/0078522 A1 betrifft eine neuronale Prothese, welche temporale Strukturen in lokalen Feldpotentialen nutzt, indem Spektralanalyse-Verfahren angewandt werden, um eine intendierte Bewegung eines Menschen vorauszusagen.

Aufgabe der Erfindung

Aufgabe der Erfindung ist es daher, Lösungen bereitzustellen, welche die aus dem Stand der Technik bekannten Nachteile zumindest teilweise vermeiden und mit welchen verbesserte bzw. schärfere Rekonstruktionsergebnisse bei einer Rekonstruktion von Gehirnaktivitäten eines Menschen ermöglicht werden.

Erfindungsgemäße Lösung

Diese Aufgabe wird erfindungsgemäß durch ein Verfahren zur Rekonstruktion von intendierten Aktivitäten aus einer ersten Repräsentation von neuronalen Signalen und durch eine Signalverarbeitungseinrichtung zur Rekonstruktion von intendierten Aktivitäten aus einer ersten Repräsentation von neuronalen Signalen nach den unabhängigen Patentansprüchen gelöst. Vorteilhafte Ausgestaltungen und Weiterbildungen der Erfindung sind in den jeweiligen abhängigen Ansprüchen angegeben.

Bereitgestellt wird demnach ein Verfahren zur Rekonstruktion von intendierten Aktivitäten aus einer ersten Repräsentation von neuronalen Signalen (S), welche indikativ für eine intendierte Aktivität ist, wobei die Signale aus einer neuronalen Aktivität des Gehirns eines Menschen abgeleitet worden sind und wobei die erste Repräsentation unter Anwendung eines vorbestimmten Verfahrens aus den neuronalen Signalen erzeugt wird,

für zweite Repräsentationen aus einer Mehrzahl vorbestimmter zweiter Repräsentationen, welche jeweils repräsentativ für eine vorbestimmte Aktivität sind und jeweils die vorbestimmte Aktivität beschreiben, wobei die vorbestimmten zweiten Repräsentationen auf der Basis von ausgeführten Aktivitäten gewonnen werden, aber nicht aus neuronalen Signalen, sondern aus technischen Aufzeichnungen von realen oder emulierten Signalen, jeweils ein Grad der Übereinstimmung zwischen der jeweiligen zweiten Repräsentation und der ersten Repräsentation gemäß einem vorbestimmten Übereinstimmungskriterium ermittelt wird, und in Abhängigkeit vom Grad der Übereinstimmung eine zweite Repräsentation der neuronalen Signale aus der Mehrzahl zweiter Repräsentationen als die für die intendierte Aktivität repräsentative zweite Repräsentation ausgewählt und die durch die ausgewählte Repräsentation repräsentierte Aktivität als die intendierte Aktivität rekonstruiert wird, wobei die Mehrzahl vorbestimmter zweiter Repräsentationen (RR) in einer Datenbank (DB) gespeichert ist,

wobei das vorbestimmte Verfahren umfasst, ein vorbestimmtes Modell anzuwenden, mit welchem die neuronale Aktivität des Gehirns in die intendierte Aktivität übersetzt wird, wobei die Gesamtheit intendierter Aktivitäten einen kontinuierlichen Parameterraum bilden,

und wobei die so rekonstruierte intendierte Aktivität von einem technischen Hilfsmittel ausgeführt wird.

Die Aktivitäten sind hier der Oberbegriff für von Menschen ausführbare Tätigkeiten, Wahrnehmungen oder gedankliche Vorstellungen. Beispiele hierfür sind Bewegungen, Sprache (genauer: Sprechen), Emotionen, Bilder (genauer Sehen), Gerüche und Tasten. Die scharfen Repräsentationen beschreiben (repräsentieren) diese Aktivitäten. Für Sprache sind dies z.B. Sonagramme, für Bewegungen z.B. Trajektkorien (der Gliedmaßen) und für Sehen Bilder.

Intendierte Aktivitäten sind die beabsichtigten oder gedachten Aktivitäten. Die erste Repräsentation, die aus den neuronalen Signalen (also aus der Gehirnaktivität des Menschen) gewonnen wird, vermag die intendierte Aktivität nur ungenau zu beschreiben, sie wird hier als indikativ für eine intendierte Aktivität bezeichnet. Sie wird hier als unscharfe Repräsentation einer intendierten Aktivität bezeichnet. Die zweiten Repräsentationen sind auf der Basis von ausgeführten Aktivitäten gewonnen, aber nicht aus neuronalen Signalen sondern aus technischen Aufzeichnungen von realen oder emulierten Aktivitäten. Sie werden hier als scharfe Repräsentationen bezeichnet, in dem Sinne, dass jeder zweiten Repräsentation eine Aktivität zugeordnet ist.

Die ersten Repräsentationen und die zweiten Repräsentationen sind gleichartig in dem Sinne, dass sie in mit denselben Methoden hinsichtlich der Aktivitäten interpretiert werden können.

Durch die Auswahl einer zweiten Repräsentation, die repräsentativ für eine vorbestimmte Aktivität ist, wird also die durch die erste Repräsentation indizierte, also nur unsicher repräsentierte Aktivität durch eine vorbestimmte Aktivität ersetzt. Diese vorbestimmte Aktivität kann von einem technischen Hilfsmittel ausgeführt werden. So können dem Hilfsmittel, etwa Lautsprecher oder Anzeigeeinrichtung, welche die vom Patienten intendierte Aktivität ausführen sollen oder zumindest dabei behilflich sein sollen, scharf rekonstruierte Aktivitäten Rekonstruktionen bereit gestellt werden. Damit wird vermieden, dass durch das Hilfsmittel unscharfe Rekonstruktionen verarbeitet werden, und so etwa an einem Lautsprecher falsche oder unverständliche Wörter oder Laute ausgegeben werden.

Das Ermitteln des Grades der Übereinstimmung gemäß dem Übereinstimmungskriterium kann ein Ermitteln einer Gesamtübereinstimmungswahrscheinlichkeit für jede zweite Repräsentation aus der Mehrzahl zweiter Repräsentationen umfassen, wobei die Gesamtübereinstimmungswahrscheinlichkeit aus einer Kombination erster Teilwahrscheinlichkeiten, welche für jede zweite Repräsentation aus der Mehrzahl zweiter Repräsentationen gebildet werden, gebildet wird, und wobei die Gesamtübereinstimmungswahrscheinlichkeit für jede zweite Repräsentation die Wahrscheinlichkeit angibt, mit der die jeweilige zweite Repräsentation der ersten Repräsentation zuordenbar ist.

Die Auswahl der zweiten Rekonstruktion kann so weiter verbessert werden.

Vorteilhafterweise wird die zweite Repräsentation mit der größten Gesamtübereinstimmungswahrscheinlichkeit ausgewählt.

Jede der ersten Teilwahrscheinlichkeiten kann mit einem Gewichtungsfaktor gewichtet werden, wobei der Gewichtungsfaktor für jede Teilwahrscheinlichkeit verschieden gewählt werden kann.

Damit ist es in vorteilhafter Weise möglich, die Gesamtübereinstimmungswahrscheinlichkeit beispielsweise situationsabhängig unterschiedlich zu ermitteln, indem je nach Situation die Teilwahrscheinlichkeiten unterschiedlich gewichtet werden.

Das Bilden der ersten Teilwahrscheinlichkeiten kann zumindest eines aus

- Ermitteln einer Teilwahrscheinlichkeit anhand der Häufigkeit der zweiten Repräsentation in der Mehrzahl vorbestimmter zweiter Repräsentationen,
- Ermitteln einer Teilwahrscheinlichkeit anhand semantischer Beziehungen der zweiten Repräsentation zu bereits ausgewählten zweiten Repräsentationen. und
- Ermitteln einer Teilwahrscheinlichkeit anhand einer Ähnlichkeit der zweiten Repräsentation zu der ersten Repräsentation

umfassen.

Es hat sich als vorteilhaft herausgestellt, wenn vor der Auswahl der zweiten Repräsentation aus der Mehrzahl zweiter Repräsentationen geprüft wird, ob die Gesamtübereinstimmungswahrscheinlichkeit der auszuwählenden zweiten Repräsentation einen vorbestimmten Schwellenwert überschreitet, wobei, wenn der vorbestimmte Schwellenwert nicht überschritten wird, die erste Repräsentation die rekonstruierte intendierte Aktivität ist.

Anhand der jeweils ermittelten Gesamtübereinstimmungswahrscheinlichkeit kann eine Anzahl zweiter Teilwahrscheinlichkeiten ermittelt werden, mit denen wiederum eine neue Gesamtübereinstimmungswahrscheinlichkeit für jede zweite Repräsentation aus der Mehrzahl zweiter Repräsentationen ermittelt wird.

Ferner kann in der Datenbank zu jeder zweiten Repräsentation eine Auswahlhäufigkeit und/oder für die in der Datenbank gespeicherten zweiten Repräsentationen eine Anzahl von Auswahlabfolgen gespeichert werden.

In der Datenbank können auch erste Repräsentationen gespeichert werden, wobei die gespeicherten ersten Repräsentationen in der Datenbank in Relation zu ausgewählten zweiten Repräsentationen gesetzt werden, wobei die in Relation zu einer zweiten Repräsentation gesetzten ersten Repräsentationen herangezogen werden, um die Ähnlichkeit der ersten Repräsentationen zu einer zweiten Repräsentationen zu ermitteln.

Die zweite Repräsentation kann durch die in Relation zu der zweiten Repräsentation gesetzten ersten Repräsentation ersetzt werden.

Die Signale können von einer außerhalb des Menschen angeordneten Mensch-Maschine-Schnittstelle abgeleitet worden sein.

Die erste Repräsentation der intendierten Aktivität wird aus den neuronalen Signalen erzeugt.

Mit einer ausgewählten zweiten Repräsentation kann ein Hilfsmittel, welches zumindest eines aus Prothese, Lautsprecher und Anzeigeeinrichtung umfasst, gesteuert werden.

Ferner wird durch die Erfindung eine Signalverarbeitungseinrichtung zur Rekonstruktion von intendierten Aktivitäten aus einer ersten Repräsentation bereitgestellt, welche mit einer Datenbank koppelbar ist und eine Schnittstelle umfasst, wobei die Schnittstelle angepasst ist, das erfindungsgemäße Verfahren auszuführen. Signale von einer Mensch-Maschine-Schnittstelle entgegenzunehmen, wobei die Signale eine neuronale Aktivität des Gehirns eines Menschen repräsentieren, und wobei die Signalverarbeitungseinrichtung angepasst ist,
eine erste Repräsentation unter Anwendung eines vorbestimmten Verfahrens aus den neuronalen Signalen zu erzeugen,
für zweite Repräsentationen aus einer Mehrzahl vorbestimmter zweiter Repräsentationen, welche jeweils repräsentativ für eine vorbestimmte Aktivität sind und jeweils die vorbestimmte Aktivität beschreiben, wobei die vorbestimmten zweiten Repräsentationen auf der Basis von ausgeführten Aktivitäten gewonnen werden, aber nicht aus neuronalen Signalen, sondern aus technischen Aufzeichnungen von realen oder emulierten Signalen, jeweils ein Grad der Übereinstimmung zwischen der jeweiligen zweiten Repräsentation und der ersten Repräsentation gemäß einem vorbestimmten Übereinstimmungskriterium zu ermitteln, und in Abhängigkeit vom Grad der Übereinstimmung eine zweite Repräsentation der neuronalen Signale aus der Mehrzahl zweiter Repräsentationen als die für die intendierte Aktivität repräsentative zweite Repräsentation auszuwählen und die durch die ausgewählte Repräsentation repräsentierte Aktivität als die intendierte Aktivität zu rekonstruieren, wobei das vorbestimmte Verfahren umfasst, ein vorbestimmtes Modell anzuwenden, mit welchem die neuronale Aktivität des Gehirns in die intendierte Aktivität übersetzt wird, wobei die Gesamtheit intendierter Aktivitäten einen kontinuierlichen Parameterraum bilden.

Vorteilhafte Ausführungsformen des Verfahrens bzw. der Einrichtung umfassen die Schritte, dass vor einem Auswählen einer zweiten Repräsentation überprüft wird, ob der Grad der Übereinstimmung ein vorgegebenes Kriterium erfüllt, und, sofern der Grad der Übereinstimmung nicht das vorgegebene Kriterium erfüllt, die intendierte Aktivität aus der ersten Repräsentation rekonstruiert wird. Diese Schritte bewirken, dass nur dann eine zweite Repräsentation aus der Mehrzahl der zweiten Repräsentationen ausgewählt wird, wenn diese wenigstens ein Mindestmaß an Übereinstimmung aufweist. Wenn dagegen unter den zweiten Repräsentationen keine passende gefunden wird, wird die intendierte Aktivität direkt aus der ersten Repräsentation ausgewählt.

Das vorbestimmte Verfahren, mit welchem die erste Repräsentation aus den neuronalen Signalen erzeugt wird, umfasst, ein vorbestimmtes Modell anzuwenden, mit welchem eine neuronale Aktivität des Gehirns in die intendierte Aktivität übersetzt wird, wobei die Gesamtheit intendierter Aktivitäten einen kontinuierlichen Parameterraum bilden. Das bedeutet, dass die Übersetzung mittels regressiver Verfahren erfolgt. Diese Übersetzung ist also keine Klassifikation, bei der ja die indikative Gehirnaktivität mit bereits bestimmter vordefinierter Repräsentationen aufgezeichneten Gehirnaktivitäten abgeglichen wird

Kurzbeschreibung der Figuren

Einzelheiten und Merkmale der Erfindung sowie konkrete Ausführungsbeispiele der Erfindung ergeben sich aus der nachfolgenden Beschreibung in Verbindung mit der Zeichnung. Es zeigt:

Fig. 1     ein Blockdiagramm zur Verdeutlichung des erfindungsgemäßen Verfahrens; und

Fig. 2     einen schematischen Aufbau einer erfindungsgemäßen Signalverarbeitungseinrichtung sowie die Koppelung der Signalverarbeitungseinrichtung mit einer erfindungsgemäßen Datenbank und einer MenschMaschine-Schnittstelle.

Detaillierte Beschreibung der Erfindung

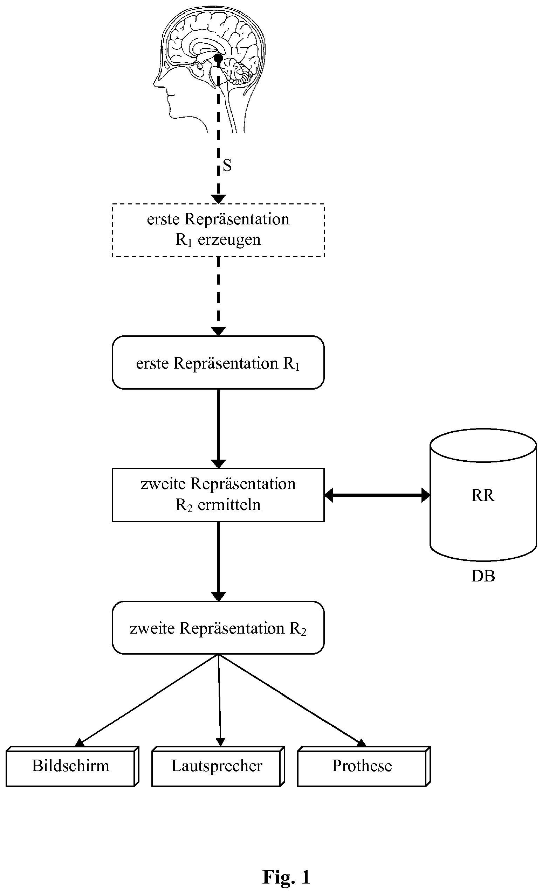

Das erfindungsgemäße Verfahren zur Rekonstruktion von Gehirnaktivitäten eines Menschen wird nachfolgend unter Bezugnahme auf **Fig. 1** näher beschrieben.

Erfindungsgemäß wird aus einer ersten Repräsentation $R_1$ von neuronalen Signalen S die entsprechende intendierte Aktivität rekonstruiert, indem aus einer Anzahl zweiter Repräsentationen RR diejenige zweite Repräsentation $R_2$ ermittelt wird, welche mit einer bestimmten Wahrscheinlichkeit der ersten Repräsentation $R_1$ entspricht. Die Mehrzahl zweiter Repräsentationen RR kann beispielsweise in einer Datenbank DB gespeichert werden, wobei vorteilhafterweise für jede Person eine Anzahl individualisierter zweiter Repräsentationen RR gespeichert werden kann.

In einer vorteilhaften Ausgestaltung der Erfindung enthält die Datenbank immer einen für die jeweilige Anwendung hinreichend vollständigen Satz an zweiten Repräsentationen RR. Das bedeutet, dass in der Datenbank DB eine Gesamtheit der für die jeweilige Anwendung benötigten, bevorzugt individuell und situativ passenden, zweite Repräsentationen RR gespeichert werden. Beispielsweise kann eine Datenbank für die sprachliche Kommunikation die in der jeweiligen Sprache vorkommenden Vokale und Konsonanten enthalten, sowie die für die Anwendung erforderlichen Wörter.

Das Erzeugen der Datenbank DB kann bzw. sollte unter Mitwirkung des jeweiligen Nutzers erfolgen. Dabei wählt der Nutzer aus der Vielzahl an möglichen Repräsentationen die von ihm bevorzugt anzuwendenden Repräsentationen aus. Ein Arzt oder ein technischer Fachmann für die jeweilige Anwendung oder für das Anlegen bzw. Erzeugen der Datenbank gibt die möglichen Repräsentationen vor und prüft die Auswahl des Nutzers auf Vollständigkeit.

Während die erste Repräsentation $R_1$, welche aus den neuronalen Signalen S erzeugt worden ist, eine hohe Unschärfe hinsichtlich der repräsentierten Aktivität aufweist, handelt es sich bei den in der Datenbank DB gespeicherten zweiten Repräsentationen RR um Repräsentationen, welche keine Unschärfe aufweisen. Die erste Repräsentation $R_1$ wird nachfolgend auch als unscharfe Repräsentation bezeichnet. Die zweite Repräsentation $R_2$ bzw. alle in der Datenbank DB gespeicherten zweiten Repräsentationen RR werden nachfolgend auch als scharfe Repräsentationen bezeichnet.

Die erste Repräsentation $R_1$ ist indikativ für bzw. beschreibt unscharf eine intendierte Aktivität eines Menschen und ist aus einem neuronalen Signal des Gehirns des betreffenden Menschen abgeleitet worden. Die intendierte Aktivität kann etwa eine gedachte Bewegung, eine gedachte Sprache (zum Beispiel Worte oder ganze Sätze), eine Emotion oder ein bildlicher Eindruck sein. Die erste bzw. unscharfe Rekonstruktion $R_1$ wird mit aus dem Stand der Technik bekannten Einrichtungen bzw. einem vorbestimmten Verfahren erzeugt. Dieses vorbestimmte Verfahren, mit welchem die erste Repräsentation aus den neuronalen Signalen erzeugt wird, umfasst, ein vorbestimmtes Modell anzuwenden, mit welchem eine neuronale Aktivität des Gehirns in die intendierte Aktivität übersetzt wird, wobei die Gesamtheit intendierter Aktivitäten einen kontinuierlichen Parameterraum bilden. Das bedeutet, dass die Übersetzung mittels regressiver Verfahren erfolgt. Diese Übersetzung ist also keine Klassifikation, bei der ja die indikative Gehirnaktivität mit bereits bestimmter vordefinierter Repräsentationen aufgezeichneten Gehirnaktivitäten abgeglichen wird

Beim Ermitteln der zweiten bzw. scharfen Repräsentation $R_2$ wird ein Grad der Übereinstimmung zwischen der ersten Repräsentation $R_1$ und der in der Datenbank DB gespeicherten zweiten Repräsentationen RR gemäß einem vorbestimmten Übereinstimmungskriterium ermittelt und in Abhängigkeit vom Grad Übereinstimmung dann eine der zweiten Repräsentationen, $R_2$, ausgewählt. In einer vorteilhaften Ausgestaltung der Erfindung wird diejenige zweite Repräsentation $R_2$ ausgewählt, welche mit der größten Wahrscheinlichkeit der ersten Repräsentation $R_1$ zugeordnet werden kann.

Die ausgewählte zweite Repräsentation $R_2$ kann dann einem Hilfsmittel zur Steuerung des Hilfsmittels zugeführt werden, wobei das Hilfsmittel in Abhängigkeit von dem Typ der zweiten Repräsentation $R_2$ beispielsweise eine Prothese, ein Lautsprecher oder ein Bildschirm sein kann. Handelt es sich bei der intendierten Aktivität beispielsweise um eine gedachte Bewegung des rechten Arms des Menschen, so ist die erste Repräsentation $R_1$ indikativ für die gedachte Bewegung. Die erste Repräsentation $R_1$ ist z.B. die Trajektorie des rechten Arms. In diesem Fall wird aus der Datenbank DB eine zweite Repräsentation $R_2$ ermittelt, welche ebenfalls indikativ für eine Bewegung ist. Die zweite Repräsentation $R_2$, ebenfalls eine Trajektorie des rechten Arms, wird dann der Prothese zugeführt, welche die Bewegung des rechten Arms ausführen soll.

Um die eine ("beste") zweite Repräsentation $R_2$ aus der Datenbank DB auszuwählen, wird für die in der Datenbank DB gespeicherten zweiten Repräsentationen RR jeweils ein Grad der Übereinstimmung zwischen der jeweiligen zweiten Repräsentation und der ersten Repräsentation $R_1$ ermittelt. In Abhängigkeit vom Grad der Übereinstimmung wird dann eine zweite Repräsentation $R_2$ aus der in der Datenbank DB gespeicherten zweiten Repräsentationen RR ausgewählt.

Der Grad der Übereinstimmung kann anhand einer Gesamtübereinstimmungswahrscheinlichkeit $P_i$ für jede zweite Repräsentation $R_i$ aus der Mehrzahl der in der Datenbank gespeicherten zweiten Repräsentation RR ermittelt werden. Die Gesamtübereinstimmungswahrscheinlichkeit $P_i$ kann aus einer Kombination erster Teilwahrscheinlichkeiten $TP_i$ gebildet werden, wobei für jede zweite Repräsentation $R_i$ der in der Datenbank DB gespeicherten zweiten Repräsentationen RR jeweils eine Anzahl erster Teilwahrscheinlichkeiten $TP_i$ erzeugt werden können.

In einer bevorzugten Ausführungsform des erfindungsgemäßen Verfahrens können folgende erste Teilwahrscheinlichkeiten $TP_i$ für die Berechnung der Gesamtübereinstimmungswahrscheinlichkeit $P_i$ berücksichtigt werden:

- erste Teilwahrscheinlichkeit $TP1_i$, welche die Häufigkeit zweiter Repräsentationen in der Mehrzahl vorbestimmter zweiter Repräsentationen RR angibt;

- erste Teilwahrscheinlichkeit $TP2_i$, welche anhand von Beziehungen der jeweiligen zweiten Repräsentation zu bereits ausgewählten zweiten Repräsentationen ermittelt wird; die Beziehungen können semantische oder historische Beziehungen sein; und

- erste Teilwahrscheinlichkeit $TP3_i$, welche die relative Ähnlichkeit der jeweiligen zweiten Repräsentation zu der ersten Repräsentation angibt.

Die ersten Teilwahrscheinlichkeiten $TP1_i$ bis $TP3_i$ können jeweils mit einem Gewichtungsfaktor $G_i$ gewichtet werden. Der Gewichtungsfaktor $G_i$ kann für jede Teilwahrscheinlichkeit verschieden gewählt werden. Die Gesamtübereinstimmungswahrscheinlichkeit $P_i$ kann beispielsweise durch Bilden des Produktes über die gewichteten Teilwahrscheinlichkeiten oder durch Bilden der Summe über die gewichteten Teilwahrscheinlichkeiten berechnet werden.

In einem anschließenden Schritt wird jene zweite Repräsentation aus den in der Datenbank DB gespeicherten zweiten Repräsentationen RR ausgewählt werden, welche die größte Gesamtübereinstimmungswahrscheinlichkeit $P_i$ zu der ersten Repräsentation $R_1$ aufweist.

Weil die in der Datenbank gespeicherten zweiten Repräsentationen RR scharfe Rekonstruktionen bzw. scharfe Repräsentationen von intendierten Aktivitäten sind, ermöglicht es dieses Verfahren erstmals unverrauschte, d.h., scharfe Rekonstruktionen von intendierten Bewegungen, Sprache, Emotionen oder bildlichen Vorstellungen einem entsprechenden Hilfsmittel zuzuführen.

Die Anwendung des erfindungsgemäßen Verfahrens wird im Folgenden anhand der Rekonstruktion von Sprache, Bildern und Bewegungen aus gedachter Sprache, Bildern bzw. Bewegungen näher erläutert.

Rekonstruktion von Sprache:

Im Falle einer Rekonstruktion von Sprache aus neuronalen Signalen sind in der Datenbank DB für die jeweilige Person individualisierte zweite Repräsentationen RR in Form von Sonagrammen der Vokale, der Konsonanten sowie ganzer Wörter oder Sätze gespeichert. Individualisiert bedeutet in diesem Zusammenhang:

- der Klang, insbesondere der einzelnen Konsonanten und Vokale ist von der jeweiligen Person selber mit der eigenen Stimme aufgenommen bzw. aufgezeichnet worden. Ist die Person nicht in der Lage selbst zu sprechen, können alte Aufnahmen bzw. Aufzeichnungen herangezogen werden. Stehen auch alte Aufnahmen nicht zur Verfügung, kann der Klang, insbesondere der einzelnen Konsonanten und Vokale anhand des Alters, des Geschlechts und gegebenenfalls weiterer Merkmale einer standardisierten Datenbank entnommen werden oder alternativ durch das System generiert werden und als Sonagramme in der Datenbank DB abgespeichert werden.

- Die in Form von Sonagrammen in der Datenbank DB abzuspeichernden Wörter wurden für bzw. durch die jeweilige Person selbst und für deren Bedürfnisse ausgewählt.

Bei der Anwendung des erfindungsgemäßen Verfahrens werden die aus der Gehirnaktivität einer Person unscharf bzw. ungenau rekonstruierten Sonagramme in genaue bzw. scharfe Sonagramme "übersetzt". Das ungenau bzw. unscharf rekonstruierte Sonagramm ist aus den Signalen S, welche aus einer neuronalen Aktivität des Gehirns der Person abgeleitet worden sind, erzeugt worden und bildet die erste Repräsentation $R_1$.

Zu dem ungenau bzw. unscharf rekonstruierten Sonagramm $R_1$ wird ein scharfes Sonagramm $R_2$ aus der Datenbank DB ausgewählt, wobei zunächst Wahrscheinlichkeiten eines scharfen Sonagramms anhand verschiedener erster Indikatoren bzw. erster Teilwahrscheinlichkeiten berechnet werden. Die ersten Teilwahrscheinlichkeiten können umfassen:

- Die Wahrscheinlichkeiten möglicher Vokale, Konsonanten bzw. Wörter, die anhand der relativen Häufigkeiten in der Datenbank DB ermittelt werden (erster Indikator 1 bzw. erste Teilwahrscheinlichkeit $TP1_i$).
- Die Wahrscheinlichkeiten möglicher Vokale, Konsonanten bzw. Wörter, die anhand semantischer Beziehungen der scharfen Sonagramme zu anderen scharfen Sonagrammen in der Datenbank DB ermittelt werden (erster Indikator 2 bzw. erste Teilwahrscheinlichkeit $TP2_i$). Hierzu kann die Datenbank DB Informationen enthalten, welche für einzelne Sprachen die Wahrscheinlichkeiten von Buchstaben, Lauten und/oder Wörtern anhand vorangegangener Buchstaben, Laute bzw. Wörter angeben.
- Die Wahrscheinlichkeiten möglicher Vokale, Konsonanten bzw. Wörter, die anhand einer Ähnlichkeit der scharfen Sonagramme zu dem unscharfen Sonagramm R1 ermittelt werden (erster Indikator 3 bzw. erste Teilwahrscheinlichkeit $TP3_i$). Hierbei kann es vorteilhaft sein, aufgrund der verschiedenen Intonationsmöglichkeiten stets die Ähnlichkeit des unscharfen Sonagramms $R_1$ zu in frequenz- und zeitskalierten scharfen Sonagrammen zu bestimmen, wobei die Frequenz die Stimmlage und die Zeit die Sprechgeschwindigkeit angeben.

Jeder der vorstehend genannten ersten Indikatoren bzw. ersten Teilwahrscheinlichkeiten gibt verschiedene Wahrscheinlichkeiten für scharfe Sonagramme, die dem unscharfen Sonagramm $R_1$ zugeordnet werden können, an.

In einem nächsten Schritt werden aus den in der Datenbank DB gespeicherten zweiten Repräsentationen RR die wahrscheinlichsten zweiten Repräsentationen bzw. scharfen Sonagramme unter Zuhilfenahme der vorstehend genannten ersten Indikatoren bzw. ersten Teilwahrscheinlichkeiten ausgewählt. Hierzu ist es vorteilhaft, jeweils für Vokale, Konsonanten und Wörter getrennt

- die ersten Indikatoren bzw. ersten Teilwahrscheinlichkeiten $TP_i$ mit einem Gewichtungsfaktor $G_i$ zu gewichten, wobei der Gewichtungsfaktor für jede Teilwahrscheinlichkeit verschieden gewählt werden kann, und
- für jede zweite Repräsentation bzw. für jedes scharfe Sonagramm in der Datenbank DB eine relative Wahrscheinlichkeit über die ersten Indikatoren bzw. über die ersten Teilwahrscheinlichkeiten, d.h., eine Gesamtübereinstimmungswahrscheinlichkeit $P_i$ zu bestimmen. Die Gesamtübereinstimmungswahrscheinlichkeit $P_i$ kann durch Kombination der ersten Indikatoren bzw. ersten Teilwahrscheinlichkeiten gebildet werden. Beispielsweise kann die Gesamtübereinstimmungswahrscheinlichkeit $P_i$ aus der Summe der gewichteten ersten Indikatoren bzw. der gewichteten ersten Teilwahrscheinlichkeiten

$$P_i = \sum_{i=1}^{n} TP_i \cdot G_i$$

oder aus dem Produkt der gewichteten ersten Teilwahrscheinlichkeiten

$$P_i = \prod_{i=1}^{n} TP_i \cdot G_i$$

bestimmt werden.

In einer vorteilhaften Ausgestaltung des erfindungsgemäßen Verfahrens können nach dem Ermitteln der relativen Wahrscheinlichkeiten bzw. Gesamtübereinstimmungswahrscheinlichkeiten $P_i$ mit Hilfe der Gesamtübereinstimmungswahrscheinlichkeiten $P_i$ zweite Indikatoren bzw. zweite Teilwahrscheinlichkeiten berechnet werden. Die zweiten Indikatoren bzw. zweiten Teilwahrscheinlichkeiten können umfassen:

- die Wahrscheinlichkeiten möglicher Wörter, die anhand der zuvor ermittelten Gesamtübereinstimmungswahrscheinlichkeiten $P_i$ der Vokale und/oder Konsonanten ermittelt werden (zweiter Indikator 4 bzw. zweite Teilwahrscheinlichkeit $TP1_j$).
- Die Wahrscheinlichkeiten möglicher Konsonanten und/oder Vokale, die anhand der zuvor ermittelten Gesamtübereinstimmungswahrscheinlichkeit $P_i$ der Vokale bzw. Konsonanten ermittelt werden (zweiter Indikator 5 bzw. zweite Teilwahrscheinlichkeit $TP2_j$).

Unter Zuhilfenahme dieser zweiten Indikatoren bzw. zweiten Teilwahrscheinlichkeiten und gegebenenfalls unter Zuhilfenahme weiterer Indikatoren bzw. Teilwahrscheinlichkeiten kann die Gesamtübereinstimmungswahrscheinlichkeit $P_i$ erneut ermittelt werden. Anhand der neu ermittelten Gesamtübereinstimmungswahrscheinlichkeit kann jene zweite Repräsentation bzw. jenes scharfe Sonagramm aus der Datenbank DB ausgewählt werden, welches die größte Gesamtübereinstimmungswahrscheinlichkeit $P_i$ aufweist. Das ausgewählte Sonagramm kann zur Ausgabe über einen Lautsprecher bereitgestellt werden.

In einer Ausgestaltung des erfindungsgemäßen Verfahrens kann es vorteilhaft sein, eine Schwellenwahrscheinlichkeit vorzusehen. Überschreitet die Gesamtübereinstimmungswahrscheinlichkeit des auszuwählenden Sonagramms die Schwellenwahrscheinlichkeit nicht, so kann anstelle eines scharfen Sonagramms aus der Datenbank DB das unscharfe Sonagramm $R_1$ zur Ausgabe über einen Lautsprecher bereitgestellt werden. Normalerweise sollte aber immer zumindest ein scharfer Konsonant, ein scharfer Vokal oder ein scharfes Phonem ausgewählt werden können. Die Schwellenwahrscheinlichkeit kann hierzu vorzugsweise angepasst werden.

Ferner kann die Datenbank DB auch Pausen und Pausenlaute bzw. emotionale Laute, etwa "Äh" oder "Haha" enthalten. Ferner kann es vorgesehen sein, in der Datenbank DB Konsonanten und/oder Vokale durch Phoneme zu ersetzen bzw. um diese zu ergänzen.

Zusätzlich zu den vorstehend genannten ersten Teilwahrscheinlichkeiten und zweiten Teilwahrscheinlichkeiten können weitere Indikatoren bzw. Teilwahrscheinlichkeiten herangezogen werden, um die jeweiligen Gesamtübereinstimmungswahrscheinlichkeiten zu bestimmen. Beispielsweise kann eine Teilwahrscheinlichkeit vorgesehen sein, welche die Nutzungshäufigkeit eines Wortes in Abhängigkeit einer Person angibt.

Gemäß einer besonders vorteilhaften Ausführungsform des erfindungsgemäßen Verfahrens kann ein Optimierungs- bzw. Kalibrierungsverfahren vorgesehen sein, mit dem die einzelnen Gewichtungsfaktoren $G_i$ angepasst bzw. optimiert werden können. Die einzelnen Gewichtungsfaktoren können auch anhand einer Bewertung durch den Anwender eingestellt bzw. angepasst werden.

Rekonstruktion von Bildern:

Im Falle einer Rekonstruktion von Bildern (visuellen Vorstellungen) aus neuronalen Signalen sind in der Datenbank DB für die jeweilige Person individualisierte Bilder als zweite Repräsentationen RR gespeichert. Vorzugsweise sind in der Datenbank DB solche Bilder gespeichert, die für die vom Anwender beabsichtigte Anwendung erforderlich bzw. erwünscht sind. Für diese Bilder sind verschiedene Repräsentationen geeignet, also z.B. Darstellungen im Ortsfrequenzraum oder in anderen Spektralzerlegungen.

Beispielsweise können für ein Kind oder eine an Autismus leidende Person, welches die Bilder zur Kommunikation verwendet, Symbole für eine einfache Kommunikation in der Datenbank DB gespeichert sein. In einem weiteren Beispiel können für eine rechtsmedizinische Untersuchung Bilder von Personen, Handlungen oder Orten in der Datenbank DB gespeichert sein. Gemäß einem weiteren Beispiel können für einen Soldaten schematisierte Lagedarstellungen oder symbolhaft kodierte Informationen als zweite Repräsentationen in der Datenbank gespeichert sein. Für einen Menschen, der die Bilder zur Arbeit verwendet, können beispielsweise technische Geräte oder Zeichenelemente als Bilder bzw. zweite Repräsentationen in der Datenbank gespeichert sein. In einem weiteren Beispiel können für eine psychologische Untersuchung Bilder von emotionalen Ausdrücken, Handlungen oder Farben als zweite Repräsentationen gespeichert sein.

Die Rekonstruktion des gedachten Bildes aus der Gehirnaktivität eines Menschen erfolgt wiederum mittels gewichteter Indikatoren bzw. gewichteter Teilwahrscheinlichkeiten, wie mit Bezug auf die Rekonstruktion von Sprache erläutert.

Hier werden wiederum zunächst die Gesamtübereinstimmungswahrscheinlichkeiten der in der Datenbank DB gespeicherten scharfen Bilder RR anhand verschiedener erster Indikatoren bzw. erster Teilwahrscheinlichkeiten berechnet. Die ersten Teilwahrscheinlichkeiten können umfassen:

- die Wahrscheinlichkeiten möglicher Bilder, die anhand deren Häufigkeit in der Datenbank DB ermittelt werden.
- Die Wahrscheinlichkeiten möglicher Bilder, die anhand semantischer Beziehungen zu bereits ausgewählten bzw. vorangegangenen Bildern ermittelt werden. Hierzu können in der Datenbank DB entsprechende Informationen gespeichert sein.
- Die Wahrscheinlichkeiten möglicher Bilder, die anhand einer Ähnlichkeit des unscharfen Bildes $R_1$ zu den in der Datenbank DB gespeicherten scharfen Bildern ermittelt werden.

Diese ersten Indikatoren bzw. ersten Teilwahrscheinlichkeiten zeigen nun verschiedene Wahrscheinlichkeiten für scharfe Bilder aus der Datenbank DB, die dem unscharfen Bild $R_1$ zugeordnet werden können.

Unter Zuhilfenahme der ersten Indikatoren bzw. ersten Teilwahrscheinlichkeiten werden die wahrscheinlichsten scharfen Bilder aus der Datenbank DB ausgewählt. Hierzu wird wiederum eine Gesamtübereinstimmungswahrscheinlichkeit ermittelt, wobei

- die ersten Indikatoren bzw. ersten Teilwahrscheinlichkeiten mit einem Gewichtungsfaktor $G_i$ gewichtet werden, wobei der Gewichtungsfaktor für jede Teilwahrscheinlichkeit verschieden gewählt werden kann; und

- für jede zweite Repräsentation RR bzw. für jedes scharfe Bild aus der Datenbank DB dessen relative Wahrscheinlichkeit über die gewichteten ersten Indikatoren bzw. über die gewichteten ersten Teilwahrscheinlichkeiten ermittelt wird. Hierbei kann wiederum die Summe oder das Produkt der gewichteten Teilwahrscheinlichkeiten herangezogen werden, wie vorstehend mit Bezug auf die Rekonstruktion von Sprache erläutert.

Bei Bedarf können weitere Indikatoren bzw. Teilwahrscheinlichkeiten zur Ermittlung der Gesamtübereinstimmungswahrscheinlichkeit herangezogen werden. Anhand der Gesamtübereinstimmungswahrscheinlichkeit wird das wahrscheinlichste scharfe Bild $R_2$ aus der Datenbank DB ausgewählt und beispielsweise zur Ausgabe auf einem Bildschirm bereitgestellt.

Ferner kann auch hier eine Schwellenwahrscheinlichkeit vorgesehen sein, welche erreicht werden muss, damit das entsprechende scharfe Bild aus der Datenbank DB ausgewählt wird. Wird diese Schwellenwahrscheinlichkeit nicht erreicht, so kann das unscharfe Bild $R_1$ zur Ausgabe auf dem Bildschirm bereitgestellt werden.

In einer vorteilhaften Ausgestaltung der Erfindung sind in der Datenbank DB auch leere Bilder als zweite Repräsentationen RR gespeichert. Ferner können weitere Indikatoren bzw. Teilwahrscheinlichkeiten vorgesehen sein, welche in die Berechnung der Gesamtübereinstimmungswahrscheinlichkeit einfließen können. Beispielsweise kann eine Teilwahrscheinlichkeit vorgesehen sein, welche die Nutzungshäufigkeit eines Bildes durch den Anwender angibt.

Auch hier kann es vorteilhaft sein, die einzelnen Gewichtungsfaktoren anhand eines Optimierungs- bzw. Kalibrierungsverfahrens einzustellen bzw. anzupassen. Dies kann auch anhand einer Bewertung durch den Anwender erfolgen.

Rekonstruktion von Bewegungen:

Bei der Rekonstruktion von Bewegungen aus neuronalen Signalen sind in der Datenbank für die jeweilige Person individualisierte Bewegungen als zweite Repräsentationen RR gespeichert. Vorteilhafterweise sind solche Bewegungen gespeichert, die für die vom Anwender beabsichtigte Anwendung notwendig bzw. erwünscht sind.

Beispielsweise können für eine schwerstgelähmte Person, die mit Hilfe einer Computermaus einen Internetbrowser steuern möchte, Bewegungstrajektorien zu den jeweils aktuell möglichen Hyperlinks, Rechts-, Links- und Doppelklick sowie Scrollen des Bildschirms nach unten und nach oben gespeichert sein. Für einen Menschen, der einen künstlichen Arm steuern möchte, kann die Datenbank Bewegungstrajektorien zu den mit Hilfe einer Kamera aktuell erfassten Objekten im greifnahen Raum sowie verschiedene Griffe, etwa Präzisionsgriff, Kraftgriff, etc. gespeichert werden. Ist beispielsweise ein Objekt gegriffen worden, kann es vorteilhaft sein, die Datenbank zu ändern bzw. nur mehr solche zweite Repräsentationen zur Auswahl zur Verfügung zu stellen, welche Bewegungstrajektorien zu möglichen Zielpunkten zum Platzieren des gegriffenen Objektes im Raum repräsentieren. In einem weiteren Beispiel können für einen Menschen, der ein Fahrzeug mit einer integrierten Kamera steuern möchte, mögliche Fahrtrichtungen, Drehungen, ein Stopp und eine Beschleunigung in der Datenbank als zweite Repräsentationen gespeichert sein.

Für die Rekonstruktion der gedachten Bewegungen aus der Gehirnaktivität eines Menschen wird eine Gesamtübereinstimmungswahrscheinlichkeit ermittelt, wie mit Bezug auf die Rekonstruktion von Sprache und Bildern erläutert.

Hierbei werden zunächst erste Indikatoren bzw. erste Teilwahrscheinlichkeiten ermittelt, aus denen wiederum eine Gesamtübereinstimmungswahrscheinlichkeit ermittelt wird. Die ersten Indikatoren bzw. ersten Teilwahrscheinlichkeiten können umfassen:

- die Wahrscheinlichkeiten möglicher Bewegungen, die anhand Häufigkeit der Bewegungen in der Datenbank DB ermittelt werden.
- Die Wahrscheinlichkeiten möglicher Bewegungen, welche anhand semantischer Beziehungen der jeweiligen zweiten Repräsentation in der Datenbank zu vorangegangenen bzw. bereits ausgewählten Bewegungen ermittelt werden. Hierzu können in der Datenbank die möglichen oder zumindest typischen Bewegungsabfolgen gespeichert werden. Diese Informationen und weitere Umweltinformationen können herangezogen werden, um die aktuell möglichen Bewegungen weiter einzuschränken. Dieser Indikator bzw. diese Teilwahrscheinlichkeit verleiht der Datenbank DB eine besondere Dynamik, weil in Abhängigkeit von der aktuellen Situation oder in Abhängigkeit von vorangegangenen Bewegungen bestimmte Bewegungen in der Datenbank DB ausgeschlossen werden können.
- Die Wahrscheinlichkeiten möglicher Bewegungen, die anhand der Ähnlichkeit der jeweiligen scharfen Bewegung in der Datenbank DB zu der unscharfen Bewegung $R_1$ ermittelt werden.

Jeder dieser ersten Indikatoren bzw. jede dieser ersten Teilwahrscheinlichkeiten gibt verschiedene Wahrscheinlichkeiten für die Zuordnung der scharfen Bewegungen zu der unscharfen Bewegung $R_1$ an.

In einem nächsten Schritt werden aus den ersten Teilwahrscheinlichkeiten bzw. ersten Indikatoren Gesamtübereinstimmungswahrscheinlichkeiten für die jeweiligen scharfen Bewegungen in der Datenbank DB ermittelt. Zum Bestimmen der Gesamtübereinstimmungswahrscheinlichkeiten werden

- die Indikatoren bzw. die Teilwahrscheinlichkeiten mit einem Gewichtungsfaktor $G_i$ gewichtet, wobei der Gewichtungsfaktor für jede Teilwahrscheinlichkeit verschieden gewählt werden kann, und
- für jede scharfe Bewegung in der Datenbank DB dessen Gesamtübereinstimmungswahrscheinlichkeit bzw. relative Wahrscheinlichkeit über die Indikatoren ermittelt. Hierzu kann wiederum die Summe der gewichteten Indikatoren bzw. gewichteten Teilwahrscheinlichkeiten oder das Produkt der gewichteten Indikatoren bzw. gewichteten Teilwahrscheinlichkeiten gebildet werden.

In der Datenbank können in einer vorteilhaften Ausführungsform der Erfindung zusätzlich zu einzelnen Bewegungen bzw. Teilbewegungen auch komplette Bewegungen, beispielsweise in Form von Trajektorien mit Ziel- und Endpunkten sowie Teilbewegungen gespeichert sein. In diesem Fall können analog zur Rekonstruktion von Sprache die Indikatoren bzw. Teilwahrscheinlichkeiten für die kompletten Bewegungen und für die Teilbewegungen zum Ermitteln der Gesamtübereinstimmungswahrscheinlichkeit herangezogen werden.

Anhand der ermittelten Gesamtübereinstimmungswahrscheinlichkeiten können dann zweite Indikatoren bzw. zweite Teilwahrscheinlichkeiten berechnet werden. Die zweiten Teilwahrscheinlichkeiten können umfassen:

- die Wahrscheinlichkeiten möglicher Bewegungen, welche anhand der zuvor ermittelten Gesamtübereinstimmungswahrscheinlichkeit der kompletten Bewegungen ermittelt werden; und
- die Wahrscheinlichkeiten möglicher Bewegungen, welche anhand der zuvor ermittelten Gesamtübereinstimmungswahrscheinlichkeiten der Teilbewegungen ermittelt werden.

Unter Zuhilfenahme der zweiten Indikatoren bzw. zweiten Teilwahrscheinlichkeiten kann die Gesamtübereinstimmungswahrscheinlichkeit neu ermittelt werden. Gegebenenfalls können noch weitere zweite Indikatoren bzw. Teilwahrscheinlichkeiten berücksichtigt werden.

In einem nächsten Schritt wird die scharfe Bewegung mit der größten Gesamtübereinstimmungswahrscheinlichkeit aus der Datenbank DB ausgewählt.

Auch bei der Rekonstruktion von Bewegungen kann es vorteilhaft sein, eine Schwellenwahrscheinlichkeit vorzusehen. Sofern die Gesamtübereinstimmungswahrscheinlichkeit der auszuwählenden scharfen Bewegung aus der Datenbank DB diese Schwellenwahrscheinlichkeit nicht überschreitet, kann die unscharfe Bewegung $R_1$ für die Steuerung etwa einer Prothese bereitgestellt werden. Vorteilhaft ist es, wenn die Schwellenwahrscheinlichkeit je nach konkreter Situation angepasst werden kann. Dies ist auch bei der Rekonstruktion von Sprache bzw. Bildern vorteilhaft.

In der Datenbank DB können auch zweite Repräsentationen von Bewegungspausen gespeichert sein. Ferner können weitere erste bzw. zweite Teilwahrscheinlichkeiten für die Berechnung der jeweiligen Gesamtübereinstimmungswahrscheinlichkeit vorgesehen sein. Beispielsweise kann eine Teilwahrscheinlichkeit vorgesehen sein, welche die Wahrscheinlichkeit einer Bewegung entsprechend der Nutzungshäufigkeit der Bewegung durch den Anwender angibt. Zudem kann es vorteilhaft sein, wenn die Gewichtungsfaktoren gemäß einem Optimierungs- bzw. Kalibrierungsverfahren angepasst werden können. Die Anpassung der Gewichtungsfaktoren kann auch durch den Anwender selbst erfolgen.

In einer vorteilhaften Ausgestaltung der Erfindung ist es auch möglich, in der Datenbank sowohl zweite Repräsentationen von Sprache als auch zweite Repräsentationen von Bildern und Bewegungen zu speichern. Dadurch ist es möglich, je nach Situation sowohl Sprache, Bilder und/oder Bewegungen aus neuronalen Signalen zu rekonstruieren und für ein entsprechendes Hilfsmittel, etwa Bildschirm, Lautsprecher oder Prothese zur Verfügung zu stellen.

Die Datenbank DB kann angepasst sein, Häufigkeiten und/oder Abfolgen der genutzten zweiten Repräsentationen (Vokale, Konsonanten, Wörter, Bilder und/oder Bewegungen) zu aktualisieren. Damit kann eine adaptive, d.h., selbstlernende Datenbank zum Speichern von zweiten Repräsentationen realisiert werden.

In einer weiteren Ausgestaltung der Datenbank DB können in dieser auch die ersten Repräsentationen $R_1$ gespeichert werden und/oder die gespeicherten unscharfen Repräsentationen $R_1$ einer oder mehreren scharfen Repräsentationen $R_2$ aus RR in der Datenbank DB zugeordnet werden. Dies hat den Vorteil, dass beispielsweise eine Ähnlichkeit zwischen einer erzeugten ersten Repräsentation $R_1$ und einer zweiten Repräsentation $R_2$ in der Datenbank DB ermittelt werden kann, wobei zunächst eine Ähnlichkeit zwischen der erzeugten ersten Repräsentation $R_1$ und einer in der Datenbank gespeicherten ersten Repräsentation ermittelt wird. Die ähnlichste in der Datenbank gespeicherte erste Repräsentation kann dann herangezogen werden, um die damit verknüpfte zweite Repräsentation in der Datenbank als zur erzeugten ersten Repräsentation $R_1$ ähnliche zweite Repräsentation auszuwählen.

Ferner kann es auch vorteilhaft sein, in der Datenbank gespeicherte zweite Repräsentationen durch erzeugte erste Repräsentationen $R_1$ zu ersetzen, sodass aus einer erzeugten unscharfen ersten Repräsentation $R_1$ eine scharfe Repräsentation $R_2$ in der Datenbank DB wird. Die Aktualisierung bzw. Anpassung der Datenbank kann wahlweise automatisch oder nur nach einer positiven Bewertung durch den Anwender durchgeführt werden. Auf diese Weise kann die Datenbank DB z.B. auf sich mit der Zeit ändernde neuronale Signale aktualisiert werden.

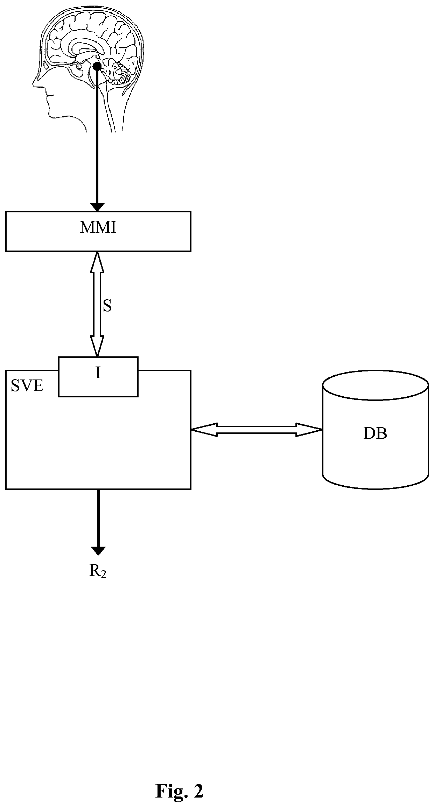

Fig. 2 zeigt ein schematisches Blockdiagramm einer erfindungsgemäßen Signalverarbeitungseinrichtung SVE zur Rekonstruktion von intendierten Aktivitäten.

Die Signalverarbeitungseinrichtung SVE umfasst eine Schnittstelle I, welche angepasst ist, Signale von einer Mensch-Maschine-Schnittstelle MMI entgegenzunehmen. Die Mensch-Maschine-Schnittstelle MMI ist vorzugsweise angepasst, aus neuronalen Aktivitäten des Menschen neuronale Signale S abzuleiten und diese an die Schnittstelle der Signalverarbeitungseinrichtung SVE zu übergeben.

Die Signalverarbeitungseinrichtung SVE ist insbesondere angepasst, das erfindungsgemäße Verfahren zur Rekonstruktion von intendierten Aktivitäten auszuführen. Hierbei ist die Signalverarbeitungseinrichtung SVE mit einer Datenbank DB gekoppelt. In einer besonderen Ausführungsform der Signalverarbeitungseinrichtung SVE kann diese auch die Datenbank DB, etwa in Form einer Embedded-Datenbank umfassen. Ferner umfasst die Signalverarbeitungseinrichtung SVE eine Schnittstelle zur Ausgabe der rekonstruierten intendierten Aktivitäten bzw. der ermittelten zweiten Repräsentation.

Die beschriebenen Ausführungsformen des Verfahrens bzw. der Einrichtung umfassen die Schritte, dass vor einem Auswählen einer zweiten Repräsentation überprüft wird, ob der Grad der Übereinstimmung ein vorgegebenes Kriterium erfüllt, und, sofern der Grad der Übereinstimmung nicht das vorgegebene Kriterium erfüllt, die intendierte Aktivität aus der ersten Repräsentation rekonstruiert wird. Diese Schritte bewirken, dass nur dann eine zweite Repräsentation aus der Mehrzahl der zweiten Repräsentationen ausgewählt wird, wenn diese wenigstens ein Mindestmaß an Übereinstimmung aufweist. Wenn dagegen unter den zweiten Repräsentationen keine passende gefunden wird, wird die intendierte Aktivität direkt aus der ersten Repräsentation ausgewählt.

**Patentansprüche**

1. Verfahren zur Rekonstruktion von intendierten Aktivitäten aus einer ersten Repräsentation ($R_1$) von neuronalen Signalen ($S$), welche indikativ für eine intendierte Aktivität ist, wobei die Signale ($S$) aus einer neuronalen Aktivität des Gehirns eines Menschen abgeleitet worden sind und wobei die erste Repräsentation ($R_1$) unter Anwendung eines vorbestimmten Verfahrens aus den neuronalen Signalen ($S$) erzeugt wird,
für zweite Repräsentationen ($R_i$) aus einer Mehrzahl vorbestimmter zweiter Repräsentationen ($RR$), welche jeweils repräsentativ für eine vorbestimmte Aktivität sind und jeweils die vorbestimmte Aktivität beschreiben, jeweils ein Grad der Übereinstimmung zwischen der jeweiligen zweiten Repräsentation ($R_i$) und der ersten Repräsentation ($R_1$) gemäß einem vorbestimmten Übereinstimmungskriterium ermittelt wird, und
in Abhängigkeit vom Grad der Übereinstimmung eine zweite Repräsentation ($R_2$) der neuronalen Signale ($S$) aus der Mehrzahl zweiter Repräsentationen ($RR$) als die für die intendierte Aktivität repräsentative zweite Repräsentation ($R_2$) ausgewählt und die durch die ausgewählte Repräsentation ($R_2$) repräsentierte Aktivität als die intendierte Aktivität rekonstruiert wird, wobei die Mehrzahl vorbestimmter zweiter Repräsentationen ($RR$) in einer Datenbank (DB) gespeichert ist,
wobei das vorbestimmte Verfahren umfasst, ein vorbestimmtes Modell anzuwenden, mit welchem die neuronale Aktivität des Gehirns in die intendierte Aktivität übersetzt wird, wobei die Gesamtheit intendierter Aktivitäten einen kontinuierlichen Parameterraum bilden, und
wobei die so rekonstruierte intendierte Aktivität von einem technischen Hilfsmittel ausgeführt wird,
**dadurch gekennzeichnet, dass** die vorbestimmten zweiten Repräsentationen ($RR$) auf der Basis von ausgeführten Aktivitäten gewonnen werden, aber nicht aus neuronalen Signalen sondern aus technischen Aufzeichnungen von realen oder emulierten Aktivitäten.

2. Verfahren nach Anspruch 1, wobei das Ermitteln des Grades der Übereinstimmung gemäß dem Übereinstimmungskriterium ein Ermitteln einer Gesamtübereinstimmungswahrscheinlichkeit ($P_i$) für jede zweite Repräsentation ($R_i$) aus der Mehrzahl zweiter Repräsentationen ($RR$) umfasst, wobei die Gesamtübereinstimmungswahrscheinlichkeit ($P_i$) aus einer Kombination erster Teilwahrscheinlichkeiten ($TP_i$), welche für jede zweite Repräsentation ($R_i$) aus der Mehrzahl zweiter Repräsentationen ($RR$) gebildet werden, gebildet wird, und wobei die Gesamtübereinstimmungswahrscheinlichkeit ($P_i$) für jede zweite Repräsentation ($R_i$) die Wahrscheinlichkeit angibt, mit der die jeweilige zweite Repräsentation ($R_i$) der ersten Repräsentation ($R_1$) zuordenbar ist,
wobei die zweite Repräsentation ($R_2$) mit der größten Gesamtübereinstimmungswahrscheinlichkeit ($P_i$) ausgewählt wird, und
wobei jede der ersten Teilwahrscheinlichkeiten ($TP_i$) mit einem Gewichtungsfaktor ($G_i$) gewichtet wird, wobei der Gewichtungsfaktor ($G_i$) für jede Teilwahrscheinlichkeit ($TP_i$) verschieden gewählt werden kann.

3. Verfahren nach Anspruch 2, wobei das Bilden der ersten Teilwahrscheinlichkeiten ($TP_i$) zumindest eines aus

    - Ermitteln einer Teilwahrscheinlichkeit ($TP1_i$) anhand der Häufigkeit der zweiten Repräsentation ($R_i$) in der Mehrzahl vorbestimmter zweiter Repräsentationen (RR),

- Ermitteln einer Teilwahrscheinlichkeit (TP2$_i$) anhand semantischer Beziehungen der zweiten Repräsentation (R$_i$) zu bereits ausgewählten zweiten Repräsentationen, und
- Ermitteln einer Teilwahrscheinlichkeit (TP3$_i$) anhand einer Ähnlichkeit der zweiten Repräsentation (R$_i$) zu der ersten Repräsentation (R$_1$) umfasst,

wobei vor der Auswahl der zweiten Repräsentation (R$_2$) aus der Mehrzahl zweiter Repräsentationen (RR) geprüft wird, ob die Gesamtübereinstimmungswahrscheinlichkeit (P$_i$) der auszuwählenden zweiten Repräsentation (R$_2$) einen vorbestimmten Schwellenwert (Ps) überschreitet, und wobei, wenn der vorbestimmte Schwellenwert (P$_S$) nicht überschritten wird, die erste Repräsentation (R$_1$) die rekonstruierte intendierte Aktivität ist, und

wobei anhand der jeweils ermittelten Gesamtübereinstimmungswahrscheinlichkeit (P$_i$) eine Anzahl zweiter Teilwahrscheinlichkeiten (TP$_j$) ermittelt werden, mit denen wiederum eine neue Gesamtübereinstimmungswahrscheinlichkeit (P$_i$) für jede zweite Repräsentation (R$_i$) aus der Mehrzahl zweiter Repräsentationen (RR) ermittelt wird.

4. Verfahren nach einem der vorherigen Ansprüche, wobei in der Datenbank zu jeder zweiten Repräsentation (R$_i$) eine Auswahlhäufigkeit und/oder für die in der Datenbank gespeicherten zweiten Repräsentationen (R$_i$) eine Anzahl von Auswahlabfolgen gespeichert ist.

5. Verfahren nach einem der vorherigen Ansprüche, wobei in der Datenbank erste Repräsentationen gespeichert werden, und wobei die gespeicherten ersten Repräsentationen in der Datenbank in Relation zu ausgewählten zweiten Repräsentationen gesetzt werden, wobei die in Relation zu einer zweiten Repräsentation (R$_i$) gesetzten ersten Repräsentationen herangezogen werden, um die Ähnlichkeit der ersten Repräsentationen (R$_1$) zu einer zweiten Repräsentationen (R$_i$) zu ermitteln.

6. Verfahren nach Anspruch 5, wobei die zweite Repräsentation durch die in Relation zu der zweiten Repräsentation (R$_i$) gesetzten ersten Repräsentation ersetzt wird.

7. Verfahren nach einem der vorhergehenden Ansprüche, wobei die ersten Repräsentationen und die zweiten Repräsentationen zumindest eines aus Bewegungen, Sprache, Emotionen, Bilder, Gerüche, und Tastsinn umfasst.

8. Verfahren nach einem der vorherigen Ansprüche, wobei die Signale (S) von einer außerhalb des Menschen angeordneten Mensch-Maschine-Schnittstelle abgeleitet worden sind.

9. Verfahren nach einem der vorhergehenden Ansprüche, wobei vor einem Auswählen einer zweiten Repräsentation (R$_2$) überprüft wird, ob der Grad der Übereinstimmung ein vorgegebenes Kriterium erfüllt, und, sofern der Grad der Übereinstimmung nicht das vorgegebene Kriterium erfüllt, die intendierte Aktivität aus der ersten Repräsentation (R$_1$) rekonstruiert wird.
tationen zu verknüpfen und/oder in der Datenbank gespeicherte zweite Repräsentationen durch die erste Repräsentation zu ersetzen.

10. Verfahren nach einem der vorhergehenden Ansprüche, wobei mit einer ausgewählten zweiten Repräsentation ein Hilfsmittel, welches zumindest eines aus Prothese, Lautsprecher und Anzeigeeinrichtung umfasst, gesteuert wird.

11. Signalverarbeitungseinrichtung (SVE) zur Rekonstruktion von intendierten Aktivitäten aus einer ersten Repräsentation (R$_1$), welche mit einer Datenbank (DB) koppelbar ist und eine Schnittstelle (I) umfasst, wobei die Schnittstelle (I) angepasst ist, Signale (S) von einer Mensch-Maschine-Schnittstelle (MMI) entgegenzunehmen, wobei die Signale (S) aus einer neuronalen Aktivität des Gehirns eines Menschen abgeleitet sind, und wobei die Signalverarbeitungseinrichtung angepasst ist,
die erste Repräsentation (R$_1$) unter Anwendung eines vorbestimmten Verfahrens aus den neuronalen Signalen (S) zu erzeugen,
für zweite Repräsentationen (R$_i$) aus einer Mehrzahl vorbestimmter zweiter Repräsentationen (RR), welche jeweils repräsentativ für eine vorbestimmte Aktivität sind und jeweils die vorbestimmte Aktivität beschreiben, jeweils ein Grad der Übereinstimmung zwischen der jeweiligen zweiten Repräsentation (R$_i$) und der ersten Repräsentation (R$_1$) gemäß einem vorbestimmten Übereinstimmungskriterium zu ermitteln, und
in Abhängigkeit vom Grad der Übereinstimmung eine zweite Repräsentation (R$_2$) der neuronalen Signale (S) aus der Mehrzahl zweiter Repräsentationen (RR) als die für die intendierte Aktivität repräsentative zweite Repräsentation (R$_2$) auszuwählen und die durch die ausgewählte Repräsentation (R$_2$) repräsentierte Aktivität als die intendierte Aktivität zu rekonstruieren, wobei das vorbestimmte Verfahren umfasst, ein vorbestimmtes Modell anzuwenden, mit welchem die neuronale Aktivität des Gehirns in die intendierte Aktivität übersetzt wird, wobei die Gesamtheit intendierter Aktivitäten einen kontinuierlichen Parameterraum bilden,
**dadurch gekennzeichnet, dass** die vorbestimmten zweiten Repräsentationen (RR) auf der Basis von ausgeführten Aktivitäten gewonnen werden, aber nicht aus neuronalen Signalen sondern aus technischen Aufzeichnungen von realen oder emulierten Aktivitäten.

12. Signalverarbeitungseinrichtung nach Anspruch 11, wobei vor einem Auswählen einer zweiten Repräsentation ($R_2$) überprüft wird, ob der Grad der Übereinstimmung ein vorgegebenes Kriterium erfüllt, und, sofern der Grad der Übereinstimmung nicht das vorgegebene Kriterium erfüllt, die intendierte Aktivität aus der ersten Repräsentation ($R_1$) rekonstruiert wird.

13. Verfahren oder Einrichtung nach einem der vorherigen Ansprüche, wobei jede der Repräsentationen jeweils ein Sonagramm darstellt, oder wobei jede der Repräsentationen jeweils eine Trajektorie einer Bewegung darstellt, oder wobei jede der Repräsentationen jeweils eine Bilddarstellung ist.

**Claims**

1. A method for reconstructing intended activities from a first representation ($R_1$) of neuronal signals (S) which is indicative of an intended activity, wherein

the signals (S) have been derived from neuronal activity of the brain of a human being, and wherein

the first representation ($R_1$) is generated from said neuronal signals (S) by using a predetermined method,

for second representations ($R_i$) from among a plurality of predetermined second representations (RR) which are each representative of a predetermined activity and respectively describe said predetermined activity, a degree of coincidence between the respective second representation ($R_i$) and the first representation ($R_1$) is determined in accordance with a predetermined criterion of coincidence, and

depending on the degree of coincidence, a second representation ($R_2$) of the neuronal signals (S) is selected from among the plurality of second representations (RR) as the second representation ($R_2$) representative of the intended activity and the activity represented by the selected representation ($R_2$) is reconstructed as the intended activity, said plurality of predetermined second representations (RR) being stored in a database (DB),

wherein the predetermined method comprises employing a predetermined model for translating the neuronal activity of the brain into the intended activity, the entirety of intended activities forming a continuous parameter space, and wherein the intended activity reconstructed in this manner is performed by some technical accessory,

**characterised in that** the predetermined second representations (RR), although derived on the basis of performed activities, are not obtained from neuronal signals but from technical records of real or emulated activities.

2. The method as claimed in claim 1, wherein determining the degree of coincidence according to the criterion of coincidence comprises determining a probability of overall coincidence ($P_i$) for each second representation ($R_i$) from among the plurality of second representations (RR), said probability of overall coincidence ($P_i$) being formed from a combination of first probabilities of partial coincidence ($TP_i$) which are formed, for each second representation ($R_i$), from a plurality of second representations (RR), and wherein the probability of overall coincidence ($P_i$) indicates, for each second representation ($R_i$), the probability with which the respective second representation ($R_i$) may be associated with the first representation ($R_1$),

wherein the second representation ($R_2$) having the greatest probability of overall coincidence ($P_i$) is selected, and

wherein each of the first probabilities of partial coincidence ($TP_i$) is weighted using a weighting factor ($G_i$), said weighting factor ($G_i$) being freely selectable so as to be different for each probability of partial coincidence ($TP_i$).

3. The method as claimed in claim 2, wherein forming the first probabilities of partial coincidence ($TP_i$) comprises at least one of:

- determining a probability of partial coincidence ($TP1_i$) by means of the frequency of occurrence of the second representation ($R_i$) in the plurality of predetermined second representations (RR),
- determining a probability of partial coincidence ($TP2_i$) by means of semantic relations between the second representation ($R_i$) and second representations that have already been selected, and
- determining a probability of partial coincidence ($TP3_i$) by means of a similarity between the second representation ($R_i$) and the first representation ($R_1$).

wherein prior to selecting the second representation ($R_2$) from among the plurality of second representations (RR), it is verified whether the probability of overall coincidence ($P_i$) of the second representation

($R_2$) to be selected does not exceed a predetermined threshold value ($P_S$), and wherein, if said predetermined threshold value ($P_S$) is not exceeded, the first representation ($R_1$) is taken to be the reconstructed intended activity, and wherein, using the individually determined probability of overall coincidence ($P_i$), a number of second probabilities of partial coincidence ($TP_j$) is determined which are then used in turn to determine a new probability of overall coincidence ($P_i$) for each second representation ($R_i$) from among the plurality of second representations (RR).

4. The method as claimed in any of the preceding claims, wherein for each second representation ($R_i$) a frequency of selection is stored in the database and/or for the second representations ($R_i$) stored in the database, a number of sequences of selection is stored.

5. The method as claimed in any of the preceding claims, wherein first representations are being stored in the database and wherein the stored first representations are related, within the database, to selected second representations, wherein the first representations that have been related to a second representation ($R_i$) are used in order to determine the degree of similarity between the first representations ($R_1$) and a second representation ($R_i$).

6. The method as claimed in claim 5, wherein the second representation is replaced by the first representation which has been related to the second representation ($R_i$).

7. The method as claimed in any of the preceding claims, wherein the first representations and the second representations comprise at least one of movements, language, emotions, images, smells, and tactile perceptions.

8. The method as claimed in any of the preceding claims, wherein the signals (S) have been derived from a man-machine interface located outside the human body.

9. The method as claimed in any of the preceding claims, wherein prior to selecting a second representation ($R_2$), it is verified whether the degree of coincidence matches a predefined criterion and, if the degree of coincidence does not match the predefined criterion, the intended activity is reconstructed from the first representation ($R_1$).

10. The method as claimed in any of the preceding claims, wherein the selected second representation is used to control an accessory comprising at least one of a prosthesis, a speaker, and a display device.

11. A signal processing device (SVE) for reconstructing intended activities from a first representation ($R_1$) which is couplable to a database (DB) and comprises an interface (I), wherein the interface (I) is adapted to receive signals (S) from a man-machine interface (MMI), wherein the signals (S) are derived from neuronal activity of a human brain, and wherein the signal processing device is adapted to

generate the first representation ($R_1$) from said neuronal signals (S) by using a predetermined method,
determine, for second representations ($R_i$) from among a plurality of predetermined second representations (RR) which are each representative of a predetermined activity and respectively describe said predetermined activity, a degree of coincidence between the respective second representation ($R_i$) and the first representation ($R_1$) in accordance with a predetermined criterion of coincidence, and
select, depending on the degree of coincidence, a second representation ($R_2$) of the neuronal signals (S) from among the plurality of second representations (RR) as the second representation ($R_2$) representative of the intended activity and to reconstruct the activity represented by the selected representation ($R_2$) as the intended activity, said predetermined method comprising employing a predetermined model by means of which the neuronal activity of the brain is translated into the intended activity, the entirety of intended activities forming a continuous parameter space, **characterised in that** the predetermined second representations (RR), although derived on the basis of performed activities, are not obtained from neuronal signals but from technical records of real or emulated activities.

12. The signal processing device as claimed in claim 11, wherein prior to selecting a second representation ($R_2$), it is verified whether the degree of coincidence matches a predefined criterion and, if the degree of coincidence does not match the predefined criterion, the intended activity is reconstructed from the first representation ($R_1$).

13. The method or device as claimed in any of the preceding claims, wherein each of the representations respectively represents a sonogram, or wherein each of the representations respectively represents a trajectory of a movement, or wherein each of the representations respectively is an image representation.

## Revendications

1. Procédé destiné à la reconstruction d'activités prévues réalisée à partir d'une première représentation ($R_1$) de signaux neuronaux (S) qui est indicative d'une activité prévue, lors duquel

les signaux (S) ont été dérivés d'une activité neuronale du cerveau d'un être humain et lors duquel la première représentation ($R_1$) est générée à partir des signaux neuronaux (S) en utilisant un procédé prédéterminé, et lors duquel pour des deuxièmes représentations ($R_i$) parmi une pluralité de deuxièmes représentations prédéterminées (RR) qui sont respectivement représentatives d'une activité prédéterminée et qui décrivent respectivement ladite activité prédéterminée est déterminé, conformément à un critère de concordance prédéterminé, respectivement un degré de concordance entre la deuxième représentation respective ($R_i$) et la première représentation ($R_1$) et une deuxième représentation ($R_2$) des signaux neuronaux (S) est sélectionnée, en fonction dudit degré de concordance, parmi la pluralité de deuxièmes représentations (RR) en tant que deuxième représentation ($R_2$) représentative de l'activité prévue, et l'activité représentée par la représentation sélectionnée ($R_2$) est reconstruite en tant qu'activité prévue, la pluralité de deuxièmes représentations prédéterminées (RR) étant stockée dans une banque de données (DB),
lors duquel ledit procédé prédéterminé comprend l'utilisation d'un modèle prédéterminé grâce auquel l'activité neuronale du cerveau est traduite en l'activité prévue, la totalité des activités prévues formant un espace de paramètres continu, et
lors duquel l'activité prévue ainsi reconstruite est exécutée par une aide technique,

**caractérisé en ce que** les deuxièmes représentations prédéterminées (RR) sont obtenues sur la base d'activités exécutées, et ce non pas à partir de signaux neuronaux mais à partir d'enregistrements techniques d'activités réelles ou émulées.

2. Procédé selon la revendication 1, lors duquel la détermination du degré de concordance conformément au critère de concordance comprend une détermination d'une probabilité de concordance totale ($P_i$) pour chaque deuxième représentation ($R_i$) parmi la pluralité de deuxièmes représentations (RR), la probabilité de concordance totale ($P_i$) étant formée à partir d'une combinaison de premières probabilités partielles ($TP_i$) qui sont formées pour chaque deuxième représentation ($R_i$) parmi la pluralité de deuxièmes représentations (RR), et la probabilité de concordance totale ($P_i$) indiquant pour chaque deuxième représentation ($R_i$) la probabilité avec laquelle la deuxième représentation respective ($R_i$) peut être attribuée à la première représentation ($R_1$),

lors duquel la deuxième représentation ($R_2$) ayant la plus grande probabilité de concordance totale ($P_i$) est sélectionnée, et
lors duquel chacune des premières probabilités partielles ($TP_i$) est pondérée avec un facteur de pondération ($G_i$), le facteur de pondération ($G_i$) pouvant être choisi de manière à être différent pour chaque probabilité partielle ($TP_i$).

3. Procédé selon la revendication 2, lors duquel la formation des premières probabilités partielles ($TP_i$) comprend au moins l'une des étapes suivantes :

- la détermination d'une probabilité partielle ($TP1_i$) à l'aide de la fréquence de la deuxième représentation ($R_i$) dans la pluralité de deuxièmes représentations prédéterminées (RR),
- la détermination d'une probabilité partielle ($TP2_i$) à l'aide de relations sémantiques entre la deuxième représentation ($R_i$) et les deuxièmes représentations qui ont déjà été sélectionnées, et
- la détermination d'une probabilité partielle ($TP3_i$) à l'aide d'une similitude entre la deuxième représentation ($R_i$) et la première représentation ($R_1$),

lors duquel il est vérifié, avant la sélection de la deuxième représentation ($R_2$) parmi la pluralité de deuxièmes représentations (RR), si la probabilité de concordance totale ($P_i$) de la deuxième représentation ($R_2$) qui doit être sélectionnée dépasse une valeur de seuil prédéterminée (Ps) et lors duquel, si ladite valeur de seuil prédéterminée ($P_s$) n'est pas dépassée, la première représentation ($R_1$) est considérée comme étant l'activité prévue reconstruite et lors duquel est déterminé, à l'aide de la probabilité de concordance totale ($P_i$) respectivement déterminée, un nombre donné de deuxièmes probabilités partielles ($TP_j$) grâce auxquelles une nouvelle probabilité de concordance totale ($P_i$) est à son tour déterminée pour chaque deuxième représentation ($R_i$) parmi la pluralité de deuxièmes représentations (RR).

4. Procédé selon l'une quelconque des revendications précédentes, lors duquel dans la banque de données est stockée une fréquence de sélection pour chaque deuxième représentation ($R_i$) et/ou est stocké un nombre donné de séquences de sélection pour les deuxièmes représentations ($R_i$) stockées dans la banque de données.

**5.** Procédé selon l'une quelconque des revendications précédentes, lors duquel des premières représentations sont stockées dans la banque de données, et lors duquel les premières représentations stockées sont mises en relation, dans ladite banque de données, avec des deuxièmes représentations sélectionnées, les premières représentations ainsi mises en relation avec une deuxième représentation ($R_i$) étant utilisées afin de déterminer le degré de similitude entre les premières représentations ($R_1$) et une deuxième représentation ($R_i$).

**6.** Procédé selon la revendication 5, lors duquel la deuxième représentation est remplacée par la première représentation qui a été mise en relation avec la deuxième représentation ($R_i$).

**7.** Procédé selon l'une quelconque des revendications précédentes, lors duquel les premières représentations et les deuxièmes représentations comprennent au moins un des éléments suivants : mouvements, langage, émotions, images, odeurs et perception tactile.

**8.** Procédé selon l'une quelconque des revendications précédentes, lors duquel les signaux (S) ont été dérivés d'une interface homme-machine disposée à l'extérieur de l'être humain.

**9.** Procédé selon l'une quelconque des revendications précédentes, lors duquel il est vérifié, avant de procéder à une sélection d'une deuxième représentation ($R_2$), si le degré de concordance remplit un critère prédéfini et, dans la mesure où le degré de concordance ne remplit pas le critère prédéfini, l'activité prévue est reconstruite à partir de la première représentation ($R_1$).

**10.** Procédé selon l'une quelconque des revendications précédentes, lors duquel une deuxième représentation sélectionnée sert à commander une aide technique qui comprend au moins l'un des éléments suivants : prothèse, haut-parleur et dispositif d'affichage.

**11.** Dispositif de traitement de signaux (SVE) destiné à la reconstruction d'activités prévues réalisée à partir d'une première représentation ($R_1$), lequel peut être couplé à une banque de données (DB) et comprend une interface (I), l'interface (I) étant adaptée pour recevoir des signaux (S) en provenance d'une interface homme-machine (MMI), les signaux (S) étant dérivés d'une activité neuronale du cerveau d'un être humain, et ledit dispositif de traitement de signaux étant adapté

pour générer la première représentation ($R_1$) à partir des signaux neuronaux (S) en utilisant un procédé prédéterminé,

pour déterminer, pour des deuxièmes représentations ($R_i$) parmi une pluralité de deuxièmes représentations prédéterminées (RR) qui sont respectivement représentatives d'une activité prédéterminée et qui décrivent respectivement ladite activité prédéterminée, respectivement un degré de concordance entre la deuxième représentation respective ($R_i$) et la première représentation ($R_1$), et ce conformément à un critère de concordance prédéterminé, et

pour sélectionner, en fonction du degré de concordance, une deuxième représentation ($R_2$) des signaux neuronaux (S) parmi la pluralité de deuxièmes représentations (RR) en tant que deuxième représentation ($R_2$) représentative de l'activité prévue, et pour reconstruire l'activité représentée par la représentation sélectionnée ($R_2$) en tant qu'activité prévue, le procédé prédéterminé comprenant l'utilisation d'un modèle prédéterminé grâce auquel l'activité neuronale du cerveau est traduite en l'activité prévue, la totalité d'activités prévues formant un espace de paramètres continu,

**caractérisé en ce que** les deuxièmes représentations prédéterminées (RR) sont obtenues sur la base d'activités exécutées, et ce non pas à partir de signaux neuronaux mais à partir d'enregistrements techniques d'activités réelles ou émulées.

**12.** Dispositif de traitement de signaux selon la revendication 11, dans lequel il est vérifié, avant de procéder à une sélection d'une deuxième représentation ($R_2$), si le degré de concordance remplit un critère prédéfini et, dans la mesure où le degré de concordance ne remplit pas le critère prédéfini, l'activité prévue est reconstruite à partir de la première représentation ($R_1$).

**13.** Procédé ou dispositif selon l'une quelconque des revendications précédentes, dans lequel chacune des représentations représente respectivement un sonagramme, ou dans lequel chacune des représentations représente respectivement une trajectoire d'un mouvement, ou dans lequel chacune des représentations est respectivement une image.

**Fig. 1**

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20100094097 A1
- US 20030078522 A1

**In der Beschreibung aufgeführte Nicht-Patentliteratur**

- **JUSTIN C. SANCHEZ et al.** Toward Brain-Computer Interfacing (Neural Information Processing). Morgan & Claypool Publishers, November 2006
- **GUIDO DORNHEGE et al.** Brain-Computer Interfaces: An international assessment of research and development trends. The MIT Press, Juli 2007
- **THEODORE W. BERGER et al.** beschrieben. Springer Netherlands, November 2008
- **ARIELI A. et al.** Dynamics of ongoing activity: explanation of the large variability in evoked cortical responses. *Science,* 27. September 1996, 1868-1871